# EUROPEAN PATENT SPECIFICATION

(11) **EP 0 953 333 B1**
(45) Date of publication and mention of the grant of the patent: **10.09.2003**
(21) Application number: 99400721.9
(22) Date of filing: 24.03.1999
(51) Int. Cl.: A61K 7/06, A61K 7/48, A61K 7/50

(54) **Cleansing composition**
Reinigungsmittel
Composition nettoyante

(30) Priority: 08.04.1998 JP 9591298
(43) Date of publication of application: 03.11.1999
(73) Proprietor: Shin-Etsu Chemical Co., Ltd., Chiyoda-ku Tokyo 100 (JP)
(72) Inventor: Nakanishi, Tetsuo, c/o Shin-Etsu Chemical Co., Ltd, Usui-gun, Gunma-ken (JP); Sakuta, Koji, c/o Shin-Etsu Chemical Co., Ltd, Usui-gun, Gunma-ken (JP)
(74) Representative: Armengaud Ainé, Alain

(56) References cited:
- EP-A- 0 426 520
- US-A- 5 165 917
- PATENT ABSTRACTS OF JAPAN vol. 018, no. 482 (C-1247), 8 September 1994 (1994-09-08) & JP 06 157236 A (KOSE CORP), 3 June 1994 (1994-06-03)

## Description

### BACKGROUND OF THE INVENTION

The present invention relates to a novel cleansing composition or, more particularly, to a cleansing composition suitable for removing oleaginous stains including an excessive amount or remnant of a makeup cosmetic composition, such as lipsticks and foundations, and sebaceous matters secreted from human skin as well as hair-protective agents to exhibit high affinity to the oleaginous staining matters and capable of giving a pleasant and refreshing feeling to the users not only during but also after use thereof.

As is usual, some types of makeup cosmetic compositions, such as lipsticks, foundations, eye shadows, mascaras and the like, contain a large amount of solid or semi-solid oleaginous ingredients so that cleansing or removing of such a cosmetic composition from human skin cannot be complete without difficulties by the use of an ordinary soap because conventional soaps have a poor dissolving power and emulsifying power to the oleaginous ingredients in the cosmetic compositions. Accordingly, use of an oil-base cleansing composition is recommended for the purpose of removing stains on the skin by the oleaginous cosmetic compositions.

As a recent trend in the cosmetic industries, various kinds of makeup cosmetic preparations have been developed with an object to improve the sustainability of the effect of makeup finish. The makeup cosmetic preparations of improved sustainability supplied to the market as suitable for use in a hot summer weather with heavy perspiration, in particular, are formulated with an oiling agent such as a cyclic organosiloxane oligomer or a polymeric resin exhibiting good film-forming behavior on the human skin. Further, hair-care treatment preparations are sometimes formulated with various ingredients including organopolysiloxane compounds, i.e. silicones, of a high degree of polymerization or highly film-forming polymeric resins with an object to protect the hair or to impart a touch feeling of tenacity or innocence of oiliness to the hair.

A problem relative to the above mentioned highly sustainable makeup cosmetic preparations and hair-care treatment compositions having a high protective effect to the hair is that removal of an excessive amount of the composition remaining on the skin or the composition inadvertently adhering to the skin can be performed only with some difficulties. For example, conventional cleansing compositions heretofore used for the purpose, which contain a non-ionic surface active agent or a polyether-modified silicone, hardly exhibit a good cleansing effect on these cosmetic and hair-care treatment compositions. Accordingly, it is eagerly desired in the industries of cosmetic preparations to develop an improved cleasing composition capable of exhibiting a high cleansing effect even on a makeup cosmetic preparation or hair-care treatment composition having high film-forming capability.

### SUMMARY OF THE INVENTION

In view of the above described problems in the modern cosmetic industries relative to the cleansing compositions, the present invention accordingly has an object to provide a novel and improved cleansing composition having good affinity to makeup cosmetic preparations and protective hair-care treatment compositions as well as to the sebum secreted from the human skin so as to rapidly and efficiently remove the stains with good appearance of the skin after cleansing along with comfortable and refreshed feeling imparted to the user not only during but also after cleansing therewith.

Thus, the cleansing composition of the present invention comprises, as a uniform blend:
(A) from 0.5 to 80% by weight of an organosilicon compound represented by the general formula

   R-(-SiR₂-O-)ₙ-SiR₃, (I)

   in which the subscript n is zero or a positive integer not exceeding 4 and each R is, independently from the others, a monovalent group selected from the group consisting of alkyl groups having 1 to 14 carbon atoms, fluorine-substituted alkyl groups having 1 to 14 carbon atoms, alkyl groups having 2 to 14 carbon atoms interrupted between adjacent carbon atoms with an ether linkage, referred to as alkyl ether groups hereinafter, cycloalkyl groups, aryl groups and aralkyl groups, of which alkyl and alkyl ether groups are preferable, at least one of the groups in a molecule denoted by R being an alkyl group or alkyl ether group having a hydroxyl group bonded to the carbon atom; and
(B) from 0.02 to 80% by weight of a non-ionic surface active agent, the total amount of the components (A) and (B) being 100% by weight or smaller.

In particular, it is preferable that the organosilicon compound as the component (A) has at least one 4-hydroxy-2-methylbutyl group as one of the groups denoted by R.

### DETAILED DESCRIPTION OF THE PREFERRED EMBODIMENTS

As is described above, the inventive cleansing composition comprises, as the essential ingredients, the components (A) and (B) each in a specified amount, of which the organosilicon compound as the component (A) is an organosilane compound, when the subscript n is zero, or an organopolysiloxane, when the subscript n is a positive integer.

The present invention has been completed on the base of an unexpected discovery that a cleansing composition containing this unique organosilicon compound in combination with a non-ionic surface active agent exhibits a cleansing effect exceeding the effect of conventional water-base cleansing compositions and has an appearance of aesthetically excellent transparency along with comfortable and refreshed feeling of use imparted to the user of the composition not only during but also after use of the cleansing composition.

The organosilicon compound as the component (A) has an effect of enhancing the cleansing power of the cleansing composition and improving the pleasant and refreshed feeling imparted to the user. In this regard, the molecular weight of the organosilicon compound should be relatively low not to exceed, though rarely a possible case, 1000 or, preferably, 500. The organosilicon compound as the component (A) having a hydroxyl-substituted alkyl or alkyl ether group has good affinity to the oleaginous staining matters to impart a high cleansing power to the composition and, different from polyether-modified silicones conventionally formulated in cleansing compositions, is free from the disadvantage of viscosity increase when mixed with water not to impart stickiness to the cleansing composition.

In the general formula (I) representing the organosilicon compound as the component (A), R, which is a group bonded to the silicon atom, is a monovalent group selected from the group consisting of alkyl groups having 1 to 14 carbon atoms, fluorine-substituted alkyl groups having 1 to 14 carbon atoms, alkyl ether group which is an alkyl groups having 2 to 14 carbon atoms and interrupted between adjacent carbon atoms by an ether linkage -C-O-C-, cycloalkyl groups, aryl groups and aralkyl groups and at least one of the groups denoted by R is an alkyl group or alkyl ether groups having a hydroxyl group bonded to the carbon atom thereof. Examples of the alkyl group include methyl, ethyl, propyl, butyl, pentyl, hexyl, heptyl, octyl, nonyl and decyl groups. Examples of the cycloalkyl group include cyclopentyl and cyclohexyl groups. Examples of the aryl group include phenyl and tolyl groups. Examples of the fluorine-substituted alkyl group include 3,3,3-trifluoropropyl and 2-(perfluorooctyl)ethyl groups. Although these groups can be contained in the organosilicon compound either singly or as a combination of two kinds or more, it is preferable that at least a half of the groups denoted by R are methyl groups.

The organosilicon compound of the general formula (I) having a hydroxyl-substituted alkyl or alkyl ether group can be synthetically prepared by a method known in the prior art. Namely, a silicon compound, i.e. silane or siloxane compound, having a hydrogen atom directly bonded to the silicon atom, such as a methyl hydrogen silane and methyl hydrogen polysiloxane, is subjected to a hydrosilation reaction with an alcohol compound having an ethylenically unsaturated carbon-carbon bond in the presence of a catalytic amount of a platinum compound such as chloroplatinic acid, alcohol-modified chloroplatinic acids and chloroplatinic acid-vinyl siloxane complexes or a similar rhodium compound as a catalyst.

The organosilicon compound having a silicon-bonded hydrogen atom mentioned above is exemplified by trimethyl silane, pentamethyl disiloxane, 1,1,3,3-tetramethyl disiloxane and 1,1,1,2,2,3,3-heptamethyl trisiloxane though not particularly limitative thereto. The alcohol compound having an ethylenically unsaturated bond in the molecule mentioned above is exemplified by allyl alcohol, pentenol-1 and isoprenol as well as monoallyl ethers of a polyhydric alcohol such as glycol compounds and glycerin.

The organosilicon compound having a hydroxyl-containing alkyl ether group can be prepared alternatively by the dehydrohalogenation reaction between an organosilicon compound having a halogenated alkyl group and an alcohol compound in the presence of a denydrohalogenating agent.

Since it is preferable that the organosilicon compound as the component (A) has a relatively small molecular weight, the subscript n, which is zero or a positive integer, in the general formula (I) should not exceed 4. The value of n is preferably 1 or 2 so that the organosilicon compound represented by the general formula (I) is a disiloxane or trisiloxane compound, respectively.

The organosilicon compound suitable for use as the component (A) is exemplified by those expressed by the following general formulas:

Me₃SiG;

Me₃Si-O-SiMe₂-G;

Me₃Si-O-SiMeG-O-SiMe₃;

and

Me₃Si-O-SiMe₂-O-SiMe₂-G,

in which Me is a methyl group and G is an alkyl or alkyl ether group having one or more of carbon-bonded hydroxyl groups in the molecule including those of the formulas:

-C₃H₆-O-CH₂-CH(OH)-CH₂-OH;

-CH₂-CHMe-CH₂-CH₂-OH;

-C₃H₆-O-CH₂-C(CH₂-OH)₂-CH₂-CH₃;

-C₃H₆-O-CH[CH₂-O-CH₂-CH(OH)-CH₂-OH]₂;

and

-C₃H₆-O-CH₂-CH₂-OH,

though not particularly limitative thereto.

The amount of the above described component (A) compounded in the inventive cleansing composition is in the range from 0.5 to 50% by weight or, preferably, from 1 to 30% by weight though widely depending on the particular forms of the cleansing composition.

The component (B), which is the other essential ingredient compounded in the inventive cleansing composition in combination with the above described organosilicon compound as the component (A), is a non-ionic surface active agent. Various types of known non-ionic surface active agents are suitable as the component (B) without particular limitations including: sorbitan fatty acid esters such as sorbitan monolaurate; glycerin fatty acid esters such as glycerin monolaurate; fatty acid esters of sucrose such as sucrose monolaurate; polyglycerin fatty acid esters; propyleneglycol fatty acid esters; polyethyleneglycol fatty acid esters; polyoxyethylene alkyl ethers such as polyoxyethylene lauryl ethers; polyoxypropylene alkyl ethers; polyoxyethylene-glycerin fatty acid esters; polyoxyethylene-polyoxypropylene glycols; polyoxyethylene alkylphenyl ethers; polyoxyethylene-polyoxypropylene glycol fatty acid esters; polyoxyethylene-sorbitan fatty acid esters; polyoxyethylene-sorbitol fatty acid esters; polyoxyethylene fatty acid esters; polyoxyethylene castor oils; polyoxyethylene hardened castor oils; polyoxyethylene phytostanol ethers; polyoxyethylene phytosterol ethers; polyoxyethylene cholestanol ethers; polyoxyethylehe cholesterol ethers; polyoxyalkylene-modified organopolysiloxanes; polyoxyalkylene-alkyl-comodified organopolysiloxanes; alkyl alkanolamides; sugar ethers; sugar amides; polyoxyethylene alkyl amines and polyoxyethylene fatty acid amides, though not particularly limitative thereto. These non-ionic surface active agents can be used either singly or as a combination of two kinds or more according to need.

The amount of the component (B) described above in the inventive cleansing composition is in the range from 0.02 to 80% by weight. The total amount of the components (A) and (B) naturally cannot exceed 100% and the balance of these essential ingredients, if any, includes various kinds of optional ingredients described below, which are acceptable on the human skin.

Namely, the inventive cleansing composition can be formulated, besides the essential components (A) and (B), with a great variety of ingredients compounded in conventional cleansing compositions depending on the particular forms of the cleansing composition. Examples of such optional ingredients include water, oiling agents, ionic surface active agents, powder materials, water-soluble compounds having a hydroxyl group in the molecule, water-soluble polymeric resins, film-forming agents, oil-soluble gelation agents, organic-modified clay minerals, natural plant resins, ultraviolet absorbers, moisturizing agents, antiseptic agents, antibacterial agents, perfumes, salts, antioxidants, pH controlling agents, chelating agents, refreshers, anti-inflammatory agents, skin beautifying agents, e.g., skin whitening agents, cell invigorating agents, rough skin improvers, blood circulation promotors, skin astringents and antiseborrheic agents, vitamins, amino acids, nucleic acids, hormones, clathrate compounds and others.

The above mentioned oiling agent can be any one of those conventionally used as an ingredient in cosmetic and toiletry preparations including solid, semi-solid and liquid ones originating in plants and animals as well as mineral oils, synthetic hydrocarbon oils, higher fatty acids, higher alcohols and esters thereof. Examples of suitable natural oleaginous materials and semi-synthetic products derived therefrom as an oiling agent include avocado oil, linseed oil, almond oil, insect wax, perilla oil, olive oil, cacao butter, kapok wax, *kaya* oil, carnauba wax, cod liver oil, candelilla wax, beef tallow, apricot kernel oil, spermaceti, hydrogenated oils, wheat germ oil, sesame oil, rice germ oil, rice bran oil, sugar cane wax, *sazanqua* oil, safflower oil, shear butter, Chinese tung oil, cinnamon oil, *jojoba* wax, shellac wax, turtle oil, soybean oil, thea oil, *tsubaki* oil, evening primrose oil, corn oil, lard, rape seed oil, Japanese tung oil, bran wax, germ oil, horse oil, persic oil, palm oil, palm kernel oil, castor oil, hydrogenated castor oil, castor oil fatty acid methyl ester, sunflower seed oil, grape seed oil, bayberry oil, jojoba oil, macadamia nut oil, beeswax, mink oil, cottonseed oil, cotton wax, montan wax, coconut oil, hydrogenated coconut oil, tri(coconut oil fatty acid) glyceride, mutton tallow, peanut oil, lanolin, reduced lanolin, lanolin alcohol, hard lanolin, lanolin acetate, lanolin fatty acid isopropyl ester, hexyl laurate, POE lanolin alcohol eters, POE lanolin alcohol acetate, lanolin fatty acid polyethyleneglycol esters. POE hydrogenated lanolin alcohol ethers and egg yolk oil.

The hydrocarbon oils mentioned above include ozokerite, squalane, squalene, ceresin, paraffin, paraffin wax, liquid paraffin, pristane, polyisobutylene, microcrystalline wax and Vaseline. The higher fatty acids include lauric acid, myristic acid, palmitic acid, stearic acid, behenic acid, undecylenic acid, oleic acid, linoleic acid, linolenic acid, arachidonic acid, eicosapentaenoic acid, docosahexanoenic acid, isostearic acid and 12-hydroxystearic acid. The higher alcohols include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, polyoxyethylene cholesterol ether and monostearyl glycerin ether. The ester oils include diisobutyl adipate, 2-hexyldecyl adipate, di-2-heptylundecyl adipate, N-alkylglycol monoisostearate, isocetyl isostearate, trimethylolpropane triisostearate, isotridecyl isononanoate, isononyl isononanoate, ethyleneglycol di-2-ethylhexanoate, cetyl 2-ethylhexanoate, trimethylolpropane tri-2-ethylhexanoate, pentaerithritol tetra-2-ethylhexanoate, cetyl octanoate, octyl dodecyl gum ester, oleyl oleate, octyldodecyl oleate, decyl oleate, neopentylglycol dicaprate, propyleneglycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerithritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate and diisostearyl malate.

The glyceride oils include acetoglyceride, glycerin triisooctanoate, glycerin triisostearate, glycerin triisopalmitate, glycerin tri-2-ethylhexanoate, glycerin monostearate, glycerin di-2-heptylundecanoate and glycerin trimyristate.

The silicone oils mentioned above include dimethylpolysiloxanes, methylphenylpolysiloxanes, methylhydrogenpolysiloxanes, octamethyl cyclotetrasiloxane, decamethyl cyclopentasiloxane, dodecamethyl cyclohexasiloxane, tetramethyl tetrahydrogen cyclotetrasiloxane, higheralcohol-modified silicones such as stearyloxysilicones and the like, higher fatty acid-modified silicones, silicone resins and silicone gums. The fluorocarbon oiling agents include perfluoro polyethers, perfluoro Decalin and perfluoro octane.

The above described oiling agents can be used either singly or as a combination of two kinds or more according to need.

The surface active agent mentioned above can be any of anionic, cationic and amphoteric ones without particular limitations. Examples of the anionic surface active agent include fatty acid soaps such as sodium stearate and triethanolamine palmitate, salts of a carboxylic acid such as alkyl ether carboxylic acids and condensation products of an amino acid and a fatty acid, alkyl sulfonate, salts of alkene sulfonic acid, sulfonic acid salts of a fatty acid ester, sulfonic acid salts of a fatty acid amide, sulfonic acid salts of a condensation product of an alkyl sulfonic acid salt and formaldehyde, salts of sulfuric acid esters, e,g., salts of an alkyl sulfate ester, salts of a secondary higher alcohol sulfuric acid ester, salts of an alkyl aryl ether sulfonic acid ester, salts of a sulfuric acid ester of a fatty acid ester, salts of a sulfuric acid ester of a fatty acid alkylolamide and Turkey red oil, salts of an alkyl phosphoric acid, salys of an ether phosphoric acid, salts of an amidophosphoric acid, salts of an alkyl aryl ether phosphoric acid and N-acylamino acid-based surface active agents. Examples of the cationic surface active agent include salts of amines such as salts of an alkylamine and fatty acid derivatives of a polyamine and an aminoalcohol, alkyl quaternary ammonium salts, aromatic quaternary ammonium salts, pyridinium salts and imidazolium salts. Examples of the amphoteric surface active agent include betaine, salts of an aminocarboxylic acid and imidazoline derivatives.

The powder material mentioned above is not particularly limitative in respects of the particle configuration which may be spherical, acicular or platelet-formed, particle diameter and particle structure which may be porous or non-porous and can be selected from a wide variety of conventional powder materials including moistureproof pigments, white pigments, colored pigments, inorganic and organic powder materials, pearlescent agents and organic dyes. Particular examples of the powder material include talc, mica, kaolin, calcium carbonate, magnesium carbonate, titanium dioxide, zinc oxide, red iron oxide, yellow iron oxide, black iron oxide, nylon powder, silk powder, urethane powder, silicone powder, titanium mica and tar dyes. These powder materials can be used either singly or as a composite of two kinds or more or can be used as such or after treatment with a silicone oil, fluorocarbon oil or surface active agent.

The water-soluble compound having a hydroxyl group in the molecule is exemplified by polyhydric alcohols including propyleneglycol, 1,3-butyleneglycol, dipropyleneglycol, isopreneglycol, glycerin, diglycerin, polyglycerins, trimethylolpropane, pntaerithritol, sorbitan, glucose, sorbitol, maltose, saccharose, trehalose, xylitol, methyl glucoside, butyl glucoside, polyoxyethylene methyl glucoside, polyoxypropylene methyl glucoside, polyoxyethyleneglycol, cellulose, carboxymethyl cellulose, carboxymethyl chitin, carageenan, hyaluronic acid and chondroitin sulfate, salts of pyrrolidone carboxylic acid, ethyl alcohol, isopropyl alcohol, butyl dialkyleneglycol monoalkyl ethers, e.g., diethyleneglycol monoethyl ether and diethyleneglycol monobutyl ether, cholesterol, sitosterol, phytosterol and lanosterol.

The water-soluble polymeric compound mentioned above is exemplified by vegetable-origin polymeric compounds such as gum arabic, tragacanth gum, galactan gum, carob gum, guar gum, karaya gum, carageenan, pectin, agar-agar, quince gum, starches of Indian corns, potatoes, wheats and others, algae colloids and locust bean gum, microbe-origin polymeric compounds such as xanthan gum, dextran, succinoglucan and pullulan, animal-origin polymeric compounds such as collagen, casein, albumin and gelatin, starch-based polymeric compounds such as carboxymethyl starch and methyl hydroxypropyl starch, cellulose-based polymeric compounds such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose and cellulose powder, alginic acid-based polymeric compounds such as sodium alginate and alginic acid ester of propyleneglycol, vinylic polymeric compounds such as polyvinyl methyl ether and carboxy divinyl polymer, polyoxyethylene-based polymeric compounds, polyoxyethylene-polyoxypropylene copolymeric compounds, acrylic polymeric compounds such as sodium polyacrylate, polyethyl acrylate and polyacrylamide, inorganic polymeric compounds such as cation polymers, bentonite, aluminum magnesium silicate and anhydrous silicic acid, and so on. Certain film-forming agents such as polyvinyl alcohol and polyvinyl pyrrolidone are also included in this category of water-soluble polymeric compounds.

The oil-soluble gelation agent mentioned above is exemplified by metal soaps such as aluminum stearate, magnesium stearate and zinc myristate, amino acid derivatives such as N-lauroyl-L-glutamate and α,γ-di-n-butyl amine, dextrin fatty acid esters such as dextrin palmitate, dextrin stearate and dextrin 2-ethylhexoate palmitate, sucrose fatty acid esters such as sucrose palmitate and sucrose stearate, benzylidene derivatives of sorbitol such as monobenzylidene sorbitol and dibenzylidene sorbitol and so on. Certain organic-modified clay minerals such as dimethylbenzyl dodecyl ammonium montmorillonite and dimethyl dioctadecyl ammonium montmorillonite are also included in this category of oil-soluble gelation agents.

The above mentioned ultraviolet absorber includes benzoic acid-based ultraviolet anbsorbers such as 4-amino benzoic acid, anthranilic acid-based ultraviolet absorbers such as methyl anthranilate, salicylic acid-based ultraviolet absorbers such as methyl salicylate, cinnamic acid-based ultraviolet absorbers such as octyl 4-methoxycinnamate, benzophenone-based ultraviolet absorbers such as 2,4-dihydroxy benzophenone, urocanic acid-based ultraviolet absorbers such as ethyl urocanate, and so on.

The antiseptic agent is exemplified by alkyl paraoxybenzoates, benzoic acid, sodium benzoate, sorbic acid, potassium sorbate and phenoxyethanol. The antimicrobial agent is exemplified by benzoic acid, salicylic acid, phenol, sorbic acid, paraoxybenzoic acid esters, p-chloro meta-cresol, hexachlorophene, benzalkonium chloride, trichloro carbanilide and phenoxy ethanol.

The antioxidant is exemplified by tocopherol, butyl hydroxyanisole (BHA) and dibutyl hydroxytoluene (BHT). The pH controlling agent is exemplified by lactic acid, citric acid, glycolic acid, succinic acid, tartaric acid, dlmalonic acid, potassium carbonate, sodium hydrogencarbonate and ammonium hydrogencarbonate. The chelating agent is exemplified by alanine, sodium edetate, sodium polyphosphate, sodium metaphosphate and phosphoric acid. The refreshing agent is exemplified by L-menthol and camphor. The anti-inflammatory agent is exemplified by alantoin, glycyrrhetinic acid and tranexamic acid and azulene.

The skin beautifying agent includes whitener agents such as placenta extract, arbutin, glutathione and saxifrage extract, cell-invigorating agents such as royal jelly, cholesterol derivatives and juvenile bovine blood extract, rough skin improvers, blood circulation promoters such as benzyl nicotinate, 2-butoxyethyl nicotinate, capsaicine, zingerone, cantharis tincture, ichthammol, caffein, tannic acid, α-borneol, tocopherol nicotinate, inocitol hexanicotinate, cyclandelate, tolazoline and acetyl choline, cepharanthine, skin astringents such as zinc oxide and tannic acid and γ-orizanol, and antiseborrheic agents such as sulfur and thianthol.

The vitamin is exemplified by vitamin A compounds such as vitamin A, retinol, retinol acetate and retinol palmitate, vitamin B₂ compounds such as riboflavin, riboflavin butyrate and flavinadenine nucleotide. vitamin B₆ compounds such as pyridoxine hydrochloride and pyridoxine dioctanoate, vitamin C compounds such as L-ascorbic acid, L-ascorbic acid dipalmitate, sodium L-ascorbic acid disulfate and dlα-tocopherol-L-ascorbic acid phosphoric acid diester dipotassium, pantothenic acid compounds such as calcium pantothenate, D-pantothenyl alcohol, pantothenyl ethyl ether and acetylpantothenyl ethyl ether, vitamin D compounds such as ergocalciferol and cholecalciferol, nicotinic acid compounds such as nicotinic acid, benzyl nicotinate and nicotinic amide, vitamin E compounds such as dl- α-tocopherol, dl- α-tocopherol acetate, dl- α-tocopherol nicotinate and dl- α-tocopherol succinate, vitamin P, biotin and so on.

The amino acid is exemplified by arginine, aspartic acid, cystine, cycteine, methionine, serine, leucine and tryptophane. The nucleic acid is exemplified by deoxy ribonucleic acid. The hormone is exemplified by estradiol and ethynyl estradiol.

The above described various compounds are each an optional additive ingredient to be admixed, either singly or as a combination of two kinds or more, with the inventive cleansing composition comprising the organosilicon compound having at least one hydroxyl-substituted alkyl or alkyl ether group in the molecule and a non-ionic surface active agent as the esential ingredients by a conventional mixing means to complete the final products of the inventive cleansing composition. The preparation form of the inventive cleansing composition is not limitative and can be in the form of a liquid, emulsion, solid, paste or gel as well as in the form of beads.

As compared with conventional cleansing compositions formulated with a non-ionic surface active agent alone or with a polyether-modified silicone alone, the inventive cleansing composition has good affinity with makeup cosmetic compositions and hair-protective compositions exhibiting high performance such as film-forming behavior as well as the sebums secreted by the human skin to rapidly accomplish the object of the cleansing treatment giving a very pleasant appearance of the skin after the cleansing treatment with a comfortable feeling of use imparted to the user not only during the cleansing treatment but also after the treatment.

In the following, the cleansing composition of the present invention is described in more detail by way of examples, which, however, never limit the scope of the invention in any way. The term of "%" appearing in the following refers always to "% by weight".

Each of the cleansing compositions prepared in Examples was formulated with one of nine hydroxyl-containing organosilicon compounds, referred to as organosilicon compounds S-1 to S-9 hereinafter, charaterized by the structural formulas given below, in which Me is a methyl group:

S-1: Me₃Si-C₃H₆OCH₂CH(OH)CH₂OH;

S-2: Me₃Si-O-SiMe₂- C₃H₆OCH₂CH(OH)CH₂OH;

S-3: Me₃Si-O-SiMe₂-CH₂CHMeCH₂CH₂OH;

S-4: Me₃Si-O-SiMe₂- C₃H₆OCH₂C(CH₂OH)₂C₂H₅

S-5: Me₃Si-O-SiMe₂-C₃H₆OCH(CH₂OCH₂CHOHCH₂OH)₂;

S-6: Me₃Si-O-SiMe₂-C₃H₆OCH₂CH₂OH;

S-7: (Me₃Si-O-)₂SiMe-C₃H₆OCH₂CH(OH)CH₂OH;

S-8: (Me₃Si-O-)₂SiMe-CH₂CHMeCH₂CH₂OH;

and

S-9: Me₃Si-O-SiMe₂-O-SiMe₂-CH₂CHMeCH₂CH₂OH.

The cleansing compositions prepared in the Examples and Comparative Examples were each subjected to evaluation tests for the items shown below by the respective testing procedures or rating criteria described there.

### I. Appearance of the composition:

The appearance of the composition was visually inspected and rated A, B and C when the composition was transparent, translucent or opaque, respectively.

### II. Organoleptic tests

Organoleptic use tests of the composition were undertaken by 50 skilled female panel members who were requested to report for the four items (II-a) to (Il-d) indicated below in five ratings of points 5, 4, 3, 2 and 1 when the use feeling for the particular item was excellent, good, fair, poor or unacceptable, respectively.
(II-a) coating spreadability of the composition over the skin
(II-b) affinitity of the composition with stain on the skin
(II-c) removability of stain from the skin
(II-d) refreshed feeling after cleansing treatment

The points given to the tested cleansing composition by each panel member were averaged for the 50 panel members and records were made for the averaged points in four ratings of A, B, C and D when the averaged point was 4.5 or larger, smaller than 4.5 but 3.5 or larger, smaller than 3.5 but 2,5 or larger, and smaller than 2.5, respectively.

### Example 1.

A cleansing composition was prepared according to the following formulation.

| | | |
|---|---|---|
| (1) | Polyoxyethylene (10 moles ethylene oxide addition) sorbitan monolaurate | 30% |
| (2) | Purified water | 50% |
| (3) | Organosilicon compound S-1 | 20% |
| | Total | 100% |

The results of the evaluation tests are shown in Table 1.

### Example 2.

The formulation of the cleansing composition was just the same as in Example 1 excepting for the replacement of the organosilicon compound S-1 with the same amount of S-2.

The results of the evaluation tests are shown also in Table 1. Example 3.

The formulation of the cleansing composition was just the same as in Example 1 excepting for the replacement of the organosilicon compound S-1 with the same amount of S-3.

The results of the evaluation tests are shown also in Table 1. Example 4.

The formulation of the cleansing composition was just the same as in Example 1 excepting for the replacement of the organosilicon compound S-1 with the same amount of S-4.

The results of the evaluation tests are shown also in Table 1. Example 5.

The formulation of the cleansing composition was just the same as in Example 1 excepting for the replacement of the organosilicon compound S-1 with the same amount of S-5.

The results of the evaluation tests are shown also in Table 1. Example 6.

The formulation of the cleansing composition was just the same as in Example 1 excepting for the replacement of the organosilicon compound S-1 with the same amount of S-6.

The results of the evaluation tests are shown also in Table 1. Example 7.

The formulation of the cleansing composition was just the same as in Example 1 excepting for the replacement of the organosilicon compound S-1 with the same amount of S-7.

The results of the evaluation tests are shown also in Table 1. Example 8.

The formulation of the cleansing composition was just the same as in Example 1 excepting for the replacement of the organosilicon compound S-1 with the same amount of S-8.

The results of the evaluation tests are shown also in Table 1. Example 9.

The formulation of the cleansing composition was just the same as in Example 1 excepting for the replacement of the organosilicon compound S-1 with the same amount of S-9.

The results of the evaluation tests are shown also in Table 1. eof as a cleansing composition are sjown also in Table 1.

### Example 10.

The formulation of the cleansing composition was just the same as in Example 1 excepting for the replacement of the polyoxyethylene sorbitan monolaurate with the same amount of a polyoxyethylene (8 moles ethylene oxide addition) oleyl ether.

The results of the evaluation tests are shown also in Table 1.

### Example 11.

The formulation of the cleansing composition was just the same as in Example 1 excepting for the replacement of the polyoxyethylene sorbitan monolaurate with the same amount of a polyoxyethylene (50 moles ethylene oxide addition) hydrogenated castor oil.

The results of the evaluation tests are shown also in Table 1. Example 12.

The formulation of the cleansing composition was just the same as in Example 1 excepting for the replacement of the polyoxyethylene sorbitan monolaurate with the same amount of a polyoxyethylene (10 moles ethylene oxide addition) monooleate.

The results of the evaluation tests are shown also in Table 1.

### Example 13.

The formulation of the cleansing composition was just the same as in Example 1 excepting for the replacement of the polyoxyethylene sorbitan monolaurate with the same amount of glycerol monolaurate.

The results of the evaluation tests are shown also in Table 1.

### Comparative Example 1.

A composition was prepared from 30% of the organosilicon compound S-1 and 70% of purified water. The results of the evaluation tests thereof as a cleansing composition are shown also in Table 1.

### Comparative Example 2.

A composition was prepared from 30% of the same polyoxyethylene sorbitan monolaurate as used in Example 1 and 70% of purified water. The results of the evaluation tests thereof as a cleansing composition are shown also in Table 1.

### Comparative Example 3.

A composition was prepared from 30% of the same polyoxyethylene sorbitan monolaurate as used in Example 1, 50% of purified water and 20% of a polyether-modified silicone as a commercial product having a molecular weight of 4500 and an HLB value of 14 (KF 6011, product by Shin-Etsu Chemical Co.). The results of the evaluation tests thereof as a cleansing composition are shown also in Table 1.

### Comparative Example 4.

A composition was prepared from 30% of the same polyoxyethylene sorbitan monolaurate as used in Example 1, 50% of purified water and 20% of another polyether-modified silicone as a commercial product having a molecular weight of 4000 and an HLB value of 10 (KF 6013, a product by Shin-Etsu Chemical Co.). The results of the evaluation tests thereof as a cleansing composition are shown also in Table 1.

### Comparative Example 5.

A composition was prepared from 30% of the same polyoxyethylene sorbitan monolaurate as used in Example 1, 50% of purified water and 20 % of a further different polyether-modified silicone as a commercial product having a molecular weight of 6000 and an HLB value of 4.5 (KF 6017, a product by Shin-Etsu Chemical Co.). The results of the evaluation tests thereof as a cleansing composition are shown also in Table 1.

**Table 1**

| Testing Item | I | Il-a | II-b | Il-c | Il-d |
|---|---|---|---|---|---|
| Example 1 | A | A | A | A | A |
| Example 2 2 | A | A | A | A | A |
| Example 3 3 | A | A | A | A | A |
| Example 4 4 | A | A | A | B | B |
| Example 5 5 | A | B | A | B | B |
| Example 6 | A | A | A | A | A |
| Example 7 7 | A | A | A | A | A |
| Example 8 8 | A | A | A | A | A |
| Example 9 9 | A | A | A | A | A |
| Example 10 | A | A | A | A | A |
| Example 11 | A | A | A | A | A |
| Example 12 | A | A | A | A | A |
| Example 13 | A | A | A | A | A |
| Comparative Example 1 | B | A | C | D | B |
| Comparative Example 2 | B | D | D | C | D |
| Comparative Example 3 | B | B | C | C | C |
| Comparative Example 4 | B | B | C | C | C |
| Comparative Example 5 | B | C | C | C | C |

### Example 14.

A cleansing composition as a makeup remover was prepared according to the following formulation.

| | | |
|---|---|---|
| (1) | Polyoxyethylene (10 moles ethylene oxide addition) sorbitan monolaurate | 10.0% |
| (2) | Organosilicon compound S-3 | 20.0% |
| (3) | Sorbitol | 10.0% |
| (4) | Carageenan | 0.5% |
| (5) | Glycerin | 5.0% |
| (6) | Antiseptic agent | q.s. |
| (7) | Perfume | q.s. |
| (8) | Purified water | (balance to 100%) |

In the first place, the ingredients (1) to (6) and (8) were mixed together to give a uniform solution, to which the ingredient (7) was added to give a makeup remover composition.

The makeup remover composition was subjected to an organoleptic evaluation test by expert panel members for the removal of a foundation on their faces. The results as reported were that the composition had excellent affinity with the foundation and the stain on the faces could be quickly removed. The composition had good and light spreadability over the skin to give a use feeling of excellent comfortableness and refreshingness during and after the removing treatment.

### Example 15.

A cleansing composition as a hair-make remover was prepared according to the following formulation.

| | | |
|---|---|---|
| (1) | Polyoxyethylene (15 moles ethylene oxide addition) Isocetyl ether | 10.0% |
| (2) | Organosilicon compound S-3 | 20.0% |
| (3) | 1,3-Butyleneglycol | 10.0% |
| (4) | Glycerin | 10.0% |
| (5) | Carageenan | 0.5% |
| (6) | Antiseptic agent | q.s. |
| (7) | Perfume | q.s. |
| (8) | Purified water | (balance to 100 %) |

In the first place, the ingredients (1) to (6) and (8) were mixed together to give a uniform solution, to which the ingredient (7) was added to give a hair-make remover composition.

The hair-make remover composition was subjected to an organoleptic evaluation test by expert panel members for cleansing of their hair as set by using a hair-make composition. The results as reported were that the composition had excellent affinity with sebaceous stain and the stain on the hair could be quickly removed. The composition had good and light spreadability over the hair to give a use feeling of excellent comfortableness and refreshingness during and after the cleansing treatment without stickiness.

### Example 16.

A face cleansing composition was prepared according to the following formulation.

| | | |
|---|---|---|
| (1) | Polyoxyethylene (6 moles ethylene oxide addition) lauryl ether | 5.0% |
| (2) | Organosilicon compound S-8 | 10.0% |
| (3) | Ethanol | 10.0% |
| (4) | Lauryl dimethylamine oxide | 2.0% |
| (5) | Propyleneglycol | 3.0% |
| (6) | Antiseptic agent | q.s. |
| (7) | Perfume | q.s. |
| (8) | Purified water | (balance to 100%) |

In the first place, the ingredients (1) to (6) and (8) were mixed together to give a uniform solution, to which the ingredient (7) was added to give a face cleansing composition.

The face cleansing composition was subjected to an organoleptic evaluation test by expert panel members for cleansing of their faces. The composition had excellent affinity with cosmetic compositions and sebaceous stain on the face and the stain on the face could be quickly removed. The composition had good and light spreadability over the skin to give a use feeling of excellent comfortableness and refreshingness during and after the face cleansing treatment.

### Example 17.

A cleansing composition as a makeup remover was prepared according to the following formulation.

| | | |
|---|---|---|
| (1) | Polyoxyethylene (6 moles ethylene oxide addition)sorbitan monolaurate | 5.0% |
| (2) | Organosilicon compound S-6 | 5.0% |
| (3) | Organosilicon compound S-8 | 15.0% |
| (4) | Ethanol | 10.0% |
| (5) | Glycerin | 2.0% |
| (6) | Dipropylene glycol | 3.0% |
| (7) | Antiseptic agent | q.s. |
| (8) | Perfume | q.s. |
| (9) | Purified water | (balance to 100%) |

In the first place, the ingredients (1) to (7) and (9) were mixed together to give a uniform solution, to which the ingredient (8) was added to give a makeup remover composition.

The makeup remover composition was subjected to an organoleptic evaluation test by expert panel members for removal of makeup compositions from their faces. The results as reported were that the composition had excellent affinity with the makeup cosmetic compositions and sebaceous stain on the face and the stain on the face could be quickly removed. The composition had good and light spreadability over the skin to give a use feeling of excellent comfortableness and refreshingness during and after the removing treatment.

## Claims

1. A cleansing composition which comprises, as a uniform blend:
(A) from 0.5 to 80% by weight of an organosilicon compound represented by the general formula
R-(-SiR₂-O-)ₙ-SiR₃,
in which the subscript n is zero or a positive integer not exceeding 4 and each R is, independently from the others, a monovalent group selected from the group consisting of alkyl groups having 1 to 14 carbon atoms, fluorine-substituted alkyl groups having 1 to 14 carbon atoms, alkyl ether groups having 2 to 14 carbon atoms, cycloalkyl groups, aryl groups and aralkyl groups, at least one of the groups in a molecule denoted by R being an alkyl group or alkyl ether group having a hydroxyl group bonded to the carbon atom; and
(B) from 0.02 to 80% by weight of a non-ionic surface active agent, the total amount of the components (A) and (B) being 100% by weight or smaller.

2. The cleansing composition as claimed in claim 1 in which the group denoted by R is an alkyl group or an alkyl ether group.

3. The cleansing composition as claimed in claim 2 in which the group denoted by R other than the alkyl group or alkyl ether group having a hydroxyl group bonded to the carbon atom is a methyl group.

4. The cleansing composition as claimed in claim 1 in which the subscript n in the general formula representing the organosilicon compound as the component (A) is 1 or 2.

5. The cleansing composition as claimed in claim 1 in which the alkyl group or alkyl ether group having a hydroxyl group in the organosilicon compound as the component (A) is selected from the group consisting of the groups expressed by the formulas:
-C₃H₆-O-CH₂-CH(OH)-CH₂-OH;
-CH₂-CHMe-CH₂-CH₂-OH;
-C₃H₆-O-CH₂-C(CH₂-OH)₂-CH₂-CH₃;
-C₃H₆-O-CH[CH₂-O-CH₂-CH(OH)-CH₂-OH]₂;
and
-C₃H₆-O-CH₂-CH₂-OH.

6. The cleansing composition as claimed in claim 5 in which the alkyl group or alkyl ether group having a hydroxyl group in the organosilicon compound as the component (A) is a 4-hydroxy-2-methylbutyl group.

7. The cleansing composition as claimed in claim 1 in which the amount of the component (A) is in the range from 1 to 30% by weight.

## Patentansprüche

1. Reinigungsmittel, das als gleichförmige Mischung enthält:
(A) 0,5 bis 80 Gew.-% einer Organosiliciumverbindung der folgenden allgemeinen Formel
R-(-SiR₂-O-)ₙ-SiR₃,
worin
der Index n für Null oder eine positive ganze Zahl von nicht mehr als 4 steht und
jede Gruppe R unabhängig von den anderen Gruppen eine monovalente Gruppe bedeutet, die ausgewählt ist aus der Gruppe bestehend aus Alkylgruppen mit 1 bis 14 Kohlenstoffatomen, fluorsubstituierten Alkylgruppen mit 1 bis 14 Kohlenstoffatomen, Alkylethergruppen mit 2 bis 14 Kohlenstoffatomen, Cycloalkylgruppen, Arylgruppen und Aralkylgruppen, wobei mindestens eine der mit R bezeichneten Gruppen in einem Molekül eine Alkylgruppe oder eine Alkylethergruppe, die ein an das Kohlenstoffatom gebundene Hydroxylgruppe aufweist, bedeutet, und
(B) 0,02 bis 80 Gew.-% eines nicht-ionischen grenzflächenaktiven Mittels, wobei die Gesamtmenge der Komponenten A und B
100 Gew.-% oder weniger ausmacht.

2. Reinigungsmittel nach Anspruch 1, bei dem die mit R bezeichnete Gruppe eine Alkylgruppe oder eine Alkylethergruppe ist.

3. Reinigungsmittel nach Anspruch 2, bei dem die mit R bezeichnete Gruppe, die keine Alkylgruppe oder Alkylethergruppe, bei der eine Hydroxylgruppe an das Kohlenstoffatom gebunden ist, bedeutet, eine Methylgruppe ist.

4. Reinigungsmittel nach Anspruch 1, bei dem der Index n in der allgemeinen Formel für die Organosiliciumverbindung als Komponente (A) für 1 oder 2 steht.

5. Reinigungsmittel nach Anspruch 1, bei dem die Alkylgruppe oder Alkylethergruppe mit einer Hydroxylgruppe in der Organosiliciumverbindung als Komponente (A) ausgewählt ist aus der Gruppe bestehend aus den nachstehenden Gruppen mit den folgenden Formeln:
-C₃H₆-O-CH₂-CH(OH)-CH₂-OH;
-CH₂-CHMe-CH₂-CH₂-OH;
-C₃H₆-O-CH₂-C(CH₂-OH)₂-CH₂-CH₃;
-C₃H₆-O-CH[CH₂-O-CH₂-CH(OH)-CH₂-OH]₂;
und
-C₃H₆-O-CH₂-CH₂-OH.

6. Reinigungsmittel nach Anspruch 5, bei dem die Alkylkgruppe oder Alkylethergruppe mit einer Hydroxylgruppe in der Siliciumverbindung als Komponente (A) eine 4-Hydroxy-2-methylbutylgruppe ist.

7. Reinigungsmittel nach Anspruch 1, bei dem die Menge an der Komponente (A) im Bereich von 1 bis 30 Gew.-% liegt.

## Revendications

1. Composition démaquillante comprenant, sous forme de mélange homogène :
(A) de 0,5 à 80 % en poids d'un composé organosilicone représenté par la formule générale
R-(-SiR₂-O-)ₙ-SiR₃,
dans laquelle l'indice n vaut zéro ou un nombre entier positif qui n'est pas supérieur à 4 et chaque R représente, indépendamment des autres, un groupe monovalent sélectionné parmi les membres du groupe constitué par des groupes alkyle comportant de 1 à 14 atomes de carbone, des groupes alkyle portant un substituant fluor et comportant de 1 à 14 atomes de carbone, des groupes alkyléther comportant de 2 à 14 atomes de carbone, des groupes cycloalkyle, des groupes aryle et des groupes aralkyle, au moins l'un des groupes de la molécule, désigné par R, étant un groupe alkyle ou alkyléther dans lequel un groupe hydroxyle est lié à l'atome de carbone ; et
(B) de 0,02 à 80 % en poids d'un tensioactif non-ionique, la proportion totale des composants (A) et (B) étant de 100 % en poids ou moins.

2. Composition démaquillante selon la revendication 1, dans laquelle le groupe représenté par R est un groupe alkyle ou un groupe alkyléther.

3. Composition démaquillante selon la revendication 2, dans laquelle le groupe représenté par R, autre que le groupe alkyle ou alkyléther dans lequel un groupe hydroxyle est lié à l'atome de carbone, est un groupe méthyle.

4. Composition démaquillante selon la revendication 1, dans laquelle l'indice n dans la formule générale représentant le composé organosilicone en tant que composant (A) vaut 1 ou 2.

5. Composition démaquillante selon la revendication 1, dans laquelle le groupe alkyle ou alkyléther, comprenant un groupe hydroxyle, du composé organosilicone en tant que composant (A) est sélectionné dans le groupe constitué par des groupes exprimés par les formules :
-C₃H₆-O-CH₂-CH(OH)-CH₂-OH ;
-CH₂-CHMe-CH₂-CH₂-OH ;
-C₃H₆-O-CH₂-C(CH₂-OH)₂-CH₂-CH₃ ;
-C₃H₆-O-CH[CH₂-O-CH₂-CH(OH)-CH₂-OH]₂ ;
et
-C₃H₆-O-CH₂-CH₂-OH.

6. Composition démaquillante selon la revendication 5, dans laquelle le groupe alkyle ou alkyléther comportant un groupe hydroxyle du composé organosilicone en tant que composant (A) est un groupe 4-hydroxy-2-méthylbutyle.

7. Composition démaquillante selon la revendication 1, dans laquelle la proportion du composant (A) est de l'ordre de 1 à 30 % en poids.
